Europäisches Patentamt

European Patent Office

Office européen des brevets

(11) Publication number: **0 149 600 B1**

# EUROPEAN PATENT SPECIFICATION
## published in accordance with Art. 158(3) EPC

(45) Date of publication of patent specification: 17.07.91   (51) Int. Cl.⁵: **A61K 7/043**

(21) Application number: 83902507.9

(22) Date of filing: 14.07.83

(86) International application number:
PCT/US83/01059

(87) International publication number:
WO 85/00288 (31.01.85 85/03)

The file contains technical information submitted
after the application was filed and not included in
this specification

(54) COMPOSITIONS FOR TREATING KERATOSIS.

(43) Date of publication of application:
31.07.85 Bulletin 85/31

(45) Publication of the grant of the patent:
17.07.91 Bulletin 91/29

(84) Designated Contracting States:
AT BE CH DE FR GB LI LU NL SE

(56) References cited:
DE-A- 2 026 305
FR-A- 471 264
US-A- 3 161 566
US-A- 3 510 554

H.Janistyn: "Handubch der Kosmetika und
Riechstoffe", vol. 3 "Die Körper-
pflegemittel", vol. 3 Die Körper-pflegemittel",
1973, Dr. Alfred Hüthig Verlag (Heildelberg,
DE) see pages 922-923 "Nagellacke", page

948 "Salicyl Collodium Nr. 2"

(73) Proprietor: SOFT SHEEN PRODUCTS, INC.
1000 East 87th Street
Chicago, IL 60619(US)

(72) Inventor: BERNSTEIN, Joel, E.
615 Brierhill Road
Deerfield, IL 60015(US)

(74) Representative: Baillie, Iain Cameron et al
c/o Ladas & Parry Isartorplatz 5
W-8000 München 2(DE)

## Description

This invention relates to an improved method of treating keratosis and is particularly directed to novel nail polish compositions for use in such improved method.

Keratosis is generally used to describe any disease of the skin characterized by an overgrowth of the spithelium. In particular, these diseases include what are commonly referred to as warts, corns and calluses. Warts are small intraepidermal growths of the skin caused by human papilloma virus and appear in children and young adults on the hands, face and feet. Corns are sharply demarcated hyperkeratotic lesions with a central core which are observed almost exclusively on the feet. Calluses are also hyperkeratotic lesions but have no central core and have a more diffuse outline. Calluses appear on the feet and hands where they may cause pain and discomfort.

Treatment of keratosis includes the use of drugs with sufficient corrosive activity so as to cause peeling of the hyperkeratotic lesion. Topical corrosive agents used in the treatment of keratosis include ascorbic acid, glacial acetic acid, lactic acid, salicylic acid, trichloroacetic acid, calcium pantothenate, zinc chloride, and podophyllum resin.

The corrosive agents are generally formulated in flexible collodion vehicles - H. Janistyn: "Handbuch der Kosmetika und Riechstoffe", vol. 3 "Die Korperpflegemittel", 1973, Dr. Alfred Huthig Verlag (Heidelberg, DE), page 948 "Salicyl Collodium Nr. 2" - or volatile solvents such as ether or alcohol. Although the collodion vehicles and solvents used in the prior art moderately reduce moisture exchange between the skin and the environment, they are not completely occlusive. It is desirable to form a complete occlusive film over the keratotic area to prevent moisture exchange between the skin and the environment, and thus increase the activity of the corrosive agent. Furthermore, the collodion vehicles and solvents used heretofore deteriorate rapidly, requiring several applications per day in order to expose the keratotic condition to the corrosive agent for a sufficient length of time.

It is an object of the invention to provide an improved method of treating keratosis with a nail polish composition which reduces the moisture exchange between the skin and the environment.

The present invention provides the use of a nail polish composition and an effective amount of at least one nontoxic and pharmaceutically acceptable corrosive agent sufficient to cause peeling of the warts, corns, or calluses as for manufacture of a medicament for relieving said warts, corns, or calluses.

The present invention also provides a composition for relieving warts, corns, and calluses comprising a nail polish composition containing an effective amount of at least one nontoxic and pharmaceutically acceptable corrosive agent, in a concentration of at least 1% by weight, sufficient to cause peeling of said warts, corns, and calluses.

In the practice of the present invention, at least one corrosive agent is incorporated into a nail polish vehicle which is applied over the area affected by keratosis. The application of the nail polish composition is a simple covering of the affected area by brushing, smearing, or painting. The composition dries quickly to form a fairly durable cover.

The present invention contemplates the use of a corrosive agent which gradually wears away the keratotic lesion by chemical action. A suitable corrosive agent is identified by its corrosive, inflammatory, and irritating action on the affected area of the skin. The corrosive agent must also be nontoxic and otherwise pharmaceutically acceptable. Exemplary corrosive agents include acids, in particular, those carboxylic acids which are pharmaceutically acceptable. In particular, the following chemicals are known corrosive agents and are preferred for use in the invention: ascorbic acid, glacial acetic acid, lactic acid, salicylic acid, trichloroacetic acid, calcium pantothenate, zinc chloride, and podophyllum resin. The present invention contemplates the use of other known corrosive agents as well.

The corrosive agent incorporated in the nail polish composition is present in an amount effective to relieve the keratotic condition with periodic application. More than one corrosive agent may be included in the nail polish composition. Preferably, each corrosive agent is present in an amount between about 1% and 40% of the total weight of the composition. A more preferred range for each corrosive agent in the composition is an amount between 1% and 20% of the total weight.

The present invention contemplates the use of any commercial nail polish, colored or clear, as a vehicle far the corrosive agent. It is critical, however, that the selected nail polish form an occlusion over the affected area when dry. Although Revlon (R.T.M.) and Cutex (R.T.M.) nail polishes are used in the examples below, these nail polishes are for illustrative purposes only and are not intended as a limitation.

Example 1

A nail polish composition useful in treating keratosis was prepared by mixing salicylic acid with Revlon (R.T.M.) clear nail polish in the amount of 1% by total weight and painting a wart on the foot of an 11 year old boy every 48 hours for 3 weeks with the composition. Unexpectedly, all signs of the wart disappeared with this treatment regimen.

## Example 2

A nail composition was prepared by mixing salicylic acid in Cutex (R.T.M.) clear nail polish in the amount of 5% by weight. The composition was applied to 5 warts on the hands of a 7 year old girl once daily for 2 weeks. Once again, the warts flattened and disappeared over this period with only one wart requiring freezing of the base.

## Example 3

A nail polish composition was prepared by mixing salicylic acid in Revlon (R.T.M.) clear nail polish in the amount of 20% by weight and the resulting composition was applied to a corn on the lateral surface of the left foot of a 37 year old woman. Unanticipated results were obtained. With similar applications made every 48 hours for 2 weeks, the corn totally disappeared.

## Example 4

A nail polish composition was prepared by mixing lactic acid into Revlon (R.T.M.) clear nail polish in the amount of 10% by weight and the resulting composition was applied once daily to a wart one cm. in diameter on the right hand of a 10 year old boy. A wart of the same size on the left hand was treated similarly with a daily application of a second nail polish composition which incorporated by weight 16% lactic acid and 16% salicylic acid in Revlon (R.T.M.) clear nail polish. After two weeks of treatment, the wart on the right hand disappeared and although the wart on the left hand nearly disappeared, some traces of the wart still remained which required additional treatment before disappearing completely.

## Example 5

A nail polish composition was prepared by mixing lactic acid in Cutex (R.T.M.) clear nail polish in the amount of 5% by weight and the resulting composition was applied 3 times weekly to a callus on the sole of the foot of a 38 year old male. Unforeseen, the callus disappeared within 10 days.

## Example 6

A nail polish composition was prepared by mixing glacial acetic acid in Revlon (R.T.M.) clear nail polish in the amount of 5% by weight and the resulting composition was applied once daily to 8 warts on the hands and elbows of a 24 year old female. Wholly unexpected results were obtained.

Within 3 weeks, no trace of the warts remained.

## Example 7

A nail polish composition was prepared by incorporating by weight 10% lactic acid and 10% salicylic acid in Revlon (R.T.M.) clear nail polish and the resulting composition was applied every other day to a large plantars wart on the sole of an 11 year old boy. After 21 days of such therapy, the wart was nearly disappeared. After freezing the base, the plantars wart never recurred.

## Example 8

A nail polish composition was prepared by mixing 15% lactic and 15% glacial acetic acid by weight in Revlon (R.T.M.) clear nail polish. The composition was applied once daily to calluses on the palms of a 38 year old man. After 1 week of such treatment the calluses disappeared.

## Example 9

A nail polish compositon was prepared by mixing 5% salicylic acid, 5% lactic acid and 5% glacial acetic acid by weight in Cutex (R.T.M.) clear nail polish and the resulting composition was applied once every 48 to 72 hours to a corn on the big toe of a 30 year old female. Unexpectedly, the corn disappeared after 2 weeks of treatment.

## Example 10

A nail polish composition was prepared by mixing 20% lactic acid and 20% glacial acetic acid by weight in Revlon (R.T.M.) clear nail polish and the resulting composition was applied once every three days to a wart on the dorsum of the right hand of a 13 year old girl. Although the skin surrounding the wart became mildly irritated, the wart disappeared within 2 weeks of treatment.

As demonstrated by these examples, this invention provides an improved method and composition for treating keratosis. The occlusion formed by the nail polish composition over the affected area reduces the moisture exchange between the skin and the environment. The durability of the nail polish composition covering the affected area allows for prolonged contact with the corrosive agent. For these reasons, the activity of the corrosive agent on the keratotic condition is increased, with fewer applications of the corrosive agent.

These examples also show that the present invention provides a method for relieving keratosis which is relatively painless, easy and convenient for both children and adults to use because of its simplicity. Furthermore, the invention is relatively inexpensive and would be affordable by the general public.

While there has been described what is at present considered to be the preferred embodiments of the invention, it will be obvious to those skilled in the art that various changes and modifications may be made therein, without departing from the invention.

## Claims

1. A composition for relieving warts, corns, and calluses comprising a nail polish composition containing an effective amount of at least one non-toxic and pharmaceutically acceptable corrosive agent, in a concentration of at least 1% by weight, sufficient to cause peeling of said warts, corns, and calluses.

2. The composition of claim 1, characterized in that each said corrosive agent is a pharmaceutically acceptable acid.

3. The composition of claim 1, characterized in that each said corrosive agent is a pharmaceutically acceptable carboxylic acid.

4. The composition of claim 1, characterized in that each said corrosive agent is selected from the group consisting of salicylic acid, lactic acid, glacial acetic acid, ascorbic acid, calcium pantothenate, podophyllum resin, and trichloroacetic acid.

## Revendications

1. Composition pour soulager l'effet des verrues, des cors et de cals comprenant une composition de vernis à ongles, contenant une quantité efficace d'au moins un agent corrosif non toxique et pharmaceutiquement acceptable en une concentration d'au moins 1% en poids qui est suffisante pour provoquer le décollage desdits cors, cals et verrues.

2. Composition selon la revendication 1, caractérisée en ce que ledit agent corrosif est un acide pharmaceutiquement acceptable.

3. Composition selon la revendication 1, caractérisée en ce que chaque agent corrosif est un acide carboxylique pharmaceutiquement acceptable.

4. Composition selon la revendication 1, caractérisée en ce que chaque agent corrosif est choisi parmi l'acide salicylique, l'acide lactique, l'acide acétique glacial, l'acide ascorbique, le pantothénate de calcium, la résine podophyllum et l'acide trichloracétique.

## Patentansprüche

1. Zusammensetzung zum Ablösen von Warzen, Hühneraugen und Schwielen mit einer Nagelpoliturzusammensetzung, die eine wirksame Menge mindestens eines ungiftigen, für pharmazeutische Zwecke geeigneten Ätzmittels in einer Konzentration von mindestens 1 Gew.-% enthält, die zum Abschälen der Warzen, Hühneraugen und Schwielen genügt.

2. Zusammensetzung nach Anspruch 1, dadurch gekennzeichnet, daß jedes der genannten Ätzmittel eine für pharmazeutische Zwecke geeignete Säure ist.

3. Zusammensetzung nach Anspruch 1, dadurch gekennzeichnet, daß jedes der genannten Ätzmittel eine für pharmazeutische Zwecke geeignete Carbonsäure ist.

4. Zusammensetzung nach Anspruch 1, dadurch gekennzeichnet, daß jedes der genannten Ätzmittel aus der Gruppe ausgewählt ist, die aus der Salicylsäure und Milchsäure, dem Eisessig, der Ascorbinsäure, dem Calciumpantothenat, Podophyllumharz und der Trichloressigsäure besteht.